(19)

Europäisches
Patentamt
European
Patent Office
Office européen
des brevets

(11) **EP 4 617 941 A1**

(12) **EUROPEAN PATENT APPLICATION**

(43) Date of publication:
**17.09.2025  Bulletin 2025/38**

(21) Application number: **25163787.2**

(22) Date of filing: **14.03.2025**

(51) International Patent Classification (IPC):
*G06F 30/23* (2020.01)      *G06F 30/27* (2020.01)
*G06N 3/044* (2023.01)      *G06F 111/10* (2020.01)
*G06F 119/14* (2020.01)

(52) Cooperative Patent Classification (CPC):
**G06F 30/23; G06F 30/27; G06N 3/044;**
G06F 2111/10; G06F 2119/14

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.03.2024  US 202418604822**

(71) Applicant: **DASSAULT SYSTEMES AMERICAS
CORP.
Waltham, MA 02451 (US)**

(72) Inventors:
• **Cojocaru, Dan
   Waltham, 02451 (US)**
• **Bi, Jing
   Waltham, 02451 (US)**

(74) Representative: **Bandpay & Greuter
11, rue Christophe Colomb
75008 Paris (FR)**

(54) **METHOD FOR ACCELERATED COMPUTATIONALLY EXPENSIVE NUMERICAL
CALIBRATIONS OF CONSTITUTIVE RESPONSE PARAMETERS USING SURROGATES**

(57)     A method for constitutive response model calibration employs neural networks based surrogate models in place of output from a corresponding finite element simulation. An application calibrates the constitutive response model upon receipt of user selections including test data output from an experiment, a finite element model simulating the experiment, a constitutive response model selection, a history output quantity, a numerical minimization algorithm, an error measure, constitutive response model parameters, an error measure for test data vs finite element simulation output, constitutive response model parameters, a parameter space sampling technique, architecture of a surrogate model architecture settings, and a training algorithm for the surrogate model training algorithm.

**Description**

FIELD OF THE INVENTION

The present invention relates to parameterization of materials, and more particularly, is related to efficiency improvements for calibrating constitutive response parameters.

BACKGROUND OF THE INVENTION

Constitutive response models (CRMs) are mathematical models used to describe the behavior of real material, structures, or interactions between structures. CRMs represent a fundamental part of finite element (FE) simulation procedures. CRMs rely on certain parameters to be specified accurately. As a simple example, when simulating a structure made of steel in elastic regime, a user may select isotropic elasticity as the constitutive response model and specify accurate values for the elastic modulus and Poisson's ratio.

While constitutive models frequently refer to material behavior, the concept of constitutive response may be expanded beyond material behaviors. For example, a mechanical part may be approximated during a finite element simulation using a simplified mathematical model, for example, by using specially formulated finite elements, e.g., spring elements, connector elements, among others. Such special elements use a special constitutive response model intended to replicate the structural behavior. Another type of CRM may be specific to modeling interactions between contacting surfaces during finite element simulations, for example, friction and wear models, and cohesive/adhesion contact models (used to simulate glued or sticky surfaces). Some constitutive response models may be complex, spanning multiple physics domains, for example, coupled electro-thermal-displacement responses.

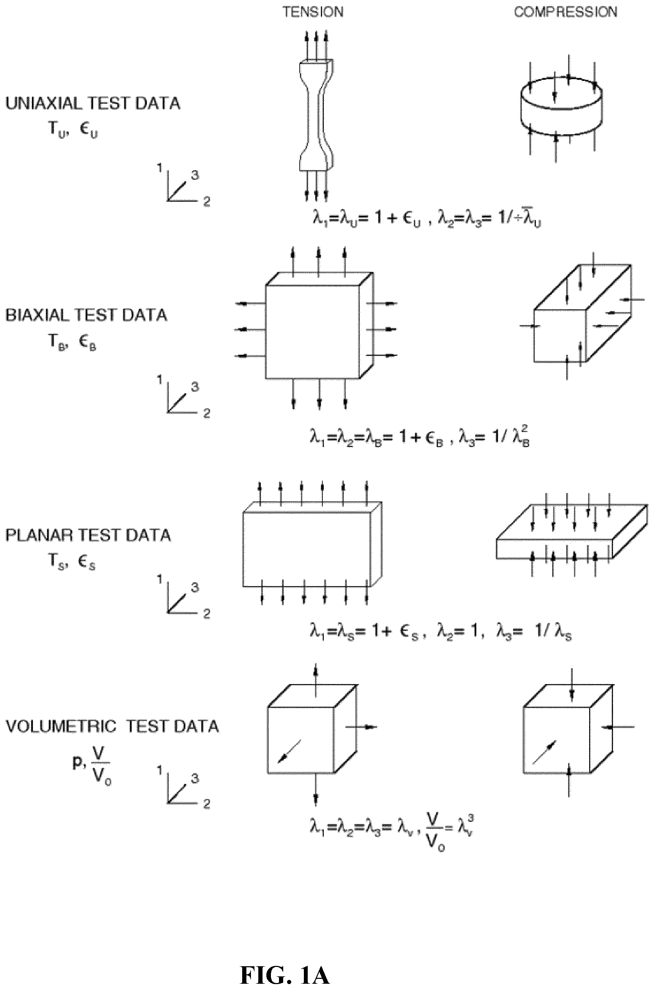

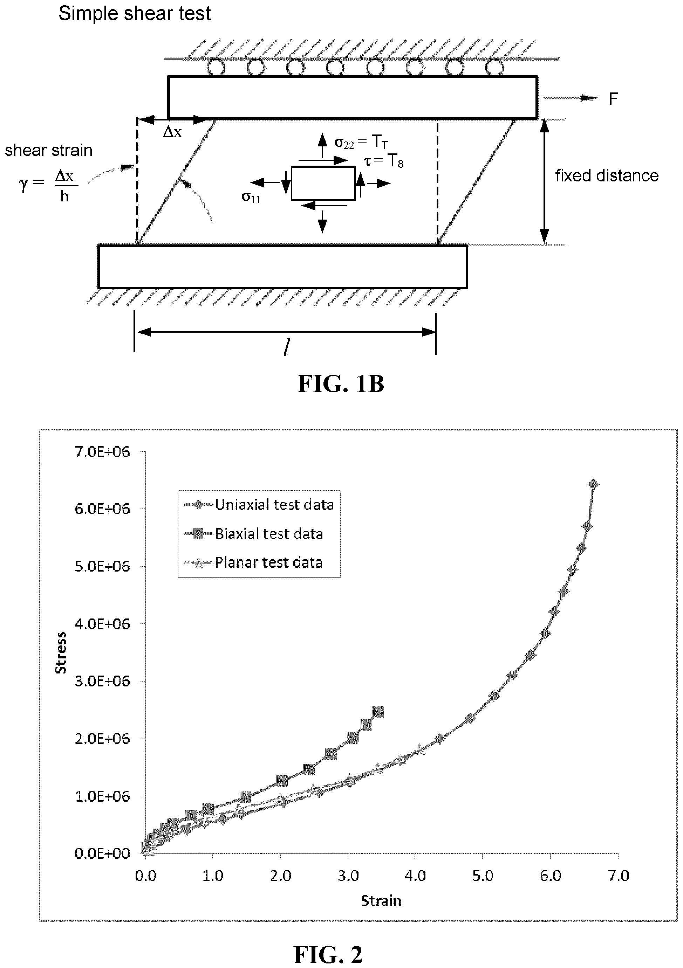

In many situations, real materials and structures need to be tested experimentally in a laboratory to obtain the parameters of the constitutive response model to be used in a finite element simulation. In general, multiple experimental setups may be used to obtain sufficient experimental data. Each experimental setup attempts to isolate the behavior of the material or structure under a particular deformation mode or a particular set of loading conditions. During each experiment, the observed behavior of the material or of the structure is recorded as a function of time, load, or deformation. Such a recording is referred to as a test data set. For example, a typical test data set may include experimentally measured stress as a function of time and/or applied strain, or may include an experimentally measured force as a function of time and/or applied displacement, among others, as illustrated by FIGS. 1A and 1B.

As shown by FIG. 1A, experiments may be used to measure and record strain-stress test data sets for certain deformation modes such as uniaxial, biaxial, planar (pure shear), volumetric and simple shear. FIG. 2 depicts an example of experimentally recorded strain-stress test data sets.

Such experimentally recorded test data sets cannot be used directly with a finite element simulation model. Instead, a typical approach is to select a suitable constitutive response model and to calibrate its parameters such that certain simulation output quantities are near agreement with the experimentally recorded test data.

With the experimental test data sets being available, normally a user will follow the following main steps in order to obtain a calibrated constitutive response:

1. Create a finite element model for each experimental setup. For example, a user may create a separate model for uniaxial deformation, biaxial deformation, etc. Such finite element models may be simple (containing a single element) or complex with multiple interacting meshed parts, each part containing a large number of elements.

2. Choose an initial guess for the parameters of the constitutive response.

3. Run the finite element simulations and collect the resulting output in a simulation output database. In some cases, the time needed to run the simulations (especially for complex models) may be very long, for example on the order of tens of hours.

4. Extract resulting data from the simulation output databases and compare the simulation output against the corresponding experimentally recorded test data.

5. If the simulation results the and the experiment results are in good agreement (i.e., the curve corresponding to the simulation output and the curve representing the experimental test data overlap or are sufficiently close to one another), the calibration process may be stopped. The agreement between the experimental test data and the simulation output is normally quantified mathematically using an error norm function such as the mean square error (MSE) function. These error norm assume a certain minimum value if the simulation output matches the experimental test data. In particular, the MSE value will be very close to zero if the simulation output is very close to the experiment test data. If the simulations results do not match the experimental results, the parameters of the constitutive response must be modified, and the simulations rerun (using the modified parameters) until a sufficiently good agreement (e.g., MSE is close to zero) is obtained.

At first, this approach was typically performed manually, but more recently a numerical minimization algorithm has been employed, using one of several known minimization algorithms. For the numerical minimization process, the minimization algorithm automatically attempts new parameter values for the constitutive response model such that the error between the simulation results and the corresponding experimental data is reduced iteratively until the simulation results are in sufficient agreement to the experimental data.

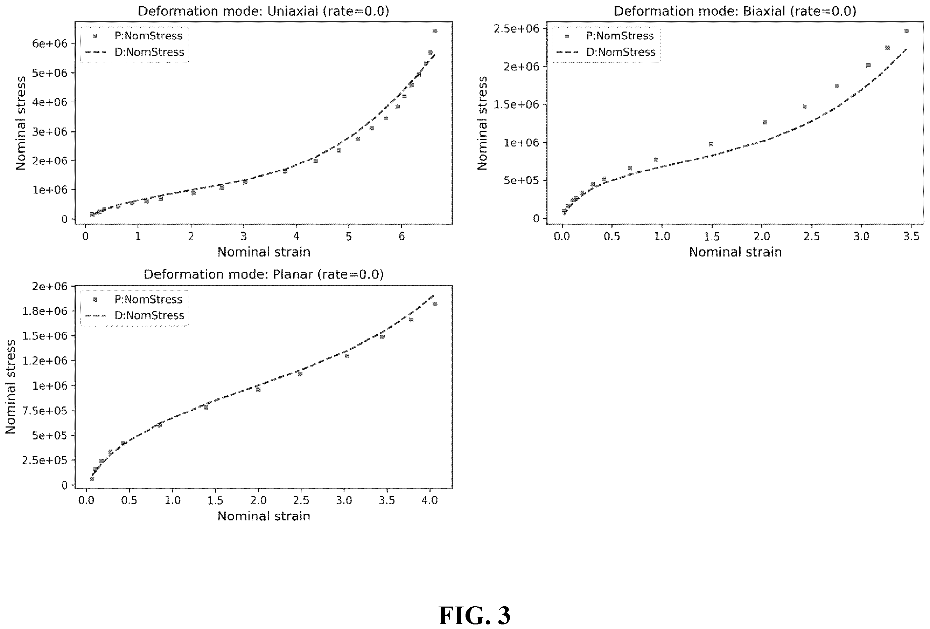

FIG. 3 shows an example of calibrated strain-stress response compared against the input test data for the uniaxial, biaxial, and planar deformation modes, where each deformation mode corresponds to a different set of loading conditions (i.e., different experimental setups, schematically represented in FIG. 1A). Herein the process of identification of the parameter values of a constitutive response by the means of a manual or automatic iterative approach using a numerical minimization algorithm is referred to as the calibration of parameters of that constitutive response. Calibration workflows employing numerical minimization algorithms may be found in existing software, including 3DX Material Calibration App.

The time needed for the automated calibration workflow is strongly dependent on the complexity and duration needed to run the finite element simulations. The computational time may be from seconds to many hours or even days and may need to employ significant computational resources. It is also possible that the user may calibrate multiple constitutive response models until a suitable one is identified, where the entire calibration process must be repeated. Therefore, there is a need in the industry to reduce the time needed to run a constitutive response calibration.

SUMMARY OF THE INVENTION

Embodiments of the present invention provide a method for accelerated computationally expensive numerical calibrations of constitutive response parameters using surrogates models. Briefly described, the present invention is directed to a method for constitutive response model calibration employing neural networks based surrogate models in place of output from a corresponding finite element simulation. An application calibrates the constitutive response model upon receipt of user selections including test data output from an experiment, a finite element model simulating the experiment, a constitutive response model selection, a history output quantity, a numerical minimization algorithm, an error measure, constitutive response model parameters, an error measure for test data vs finite element simulation output, constitutive response model parameters, a parameter space sampling technique, architecture of a surrogate model architecture settings, and a training algorithm for the surrogate model training algorithm.

Other systems, methods and features of the present invention will be or become apparent to one having ordinary skill in the art upon examining the following drawings and detailed description. It is intended that all such additional systems, methods, and features be included in this description, be within the scope of the present invention and protected by the accompanying claims.

BRIEF DESCRIPTION OF THE DRAWINGS

The patent or application file contains at least one drawing executed in color. Copies of this patent or patent application publication with color drawing(s) will be provided by the Office upon request and payment of the necessary fee.

The accompanying drawings are included to provide a further understanding of the invention, and are incorporated in and constitute a part of this specification. The components in the drawings are not necessarily to scale, emphasis instead being placed upon clearly illustrating the principles of the present invention. The drawings illustrate embodiments of the invention and, together with the description, serve to explain the principles of the invention.

FIG. 1A is a diagram of typical experiments to provide experimentally measured stress as a function of time and/or applied strain, or an experimentally measured force as a function of time and/or applied displacement.

FIG. 1B is a diagram of an experiments to provide experimentally measured shear stress.

FIG. 2 is a plot overlaying uniaxial, biaxial, and planar test data for stress vs. strain.

FIG. 3 shows individual plots of experimental test data and simulation responses corresponding to three deformation modes: uniaxial, biaxial, and planar.

FIG. 4 is a flowchart of an exemplary first method embodiment.

FIG. 5 is a flowchart of a detail of the exemplary first method embodiment of FIG. 4.

FIG 6A is a screenshot showing an example of importing experimental test data under the first embodiment.

FIG. 6B is a screenshot of an example of user imported experimental test data sets into the 3DX Material Calibration App.

FIG. 6C is a plot showing selected test data during the import process, here plotting experimental Nominal Stress as a function of Time.

FIG. 6D shows imported experimental test data for editing and display after import into the app.

FIG. 6E shows multiple experimental test data sets plotted inside the app. After import, the user may choose which test data sets to be used for a calibration.

FIG. 7A shows an example of a set of three finite element models used together during the same calibration of a plasticity with damage constitutive response model.

FIG. 7B shows an example of an Abaqus finite element model used for simulating a double cantilever beam experiment.

FIG. 7C shows an example of a set of three finite element models used together during the same calibration of an anisotropic hyper elastic constitutive response.

FIG. 7D shows an exemplary set of two finite element models used together for the calibration of a connector behavior.

FIG. 8 is a screenshot showing an exemplary list of constitutive response models for user selection.

FIG. 9 is an annotated screenshot showing an example calibration setup using three finite element models.

FIG. 10 is a screenshot illustrating user selection of one of several available numerical minimizations algorithms to be used for calibration.

FIG. 11 is a screenshot illustrating how the user can select one of the available error measures.

FIG. 12 is a screenshot showing an exemplary scenario of a user selected constitutive material model.

FIG. 13 is a screenshot showing an exemplary scenario where the user is presented options to modify default settings relating to initial sampling of the parameter space.

FIG. 14 highlights a portion of the screenshot of FIG. 13 indicating a user selection of settings regarding architecture of a surrogate model to be trained to replicate the finite element output.

FIG. 15 is a screenshot and plot comparing the FE responses using calibrated parameters to the experimental test data.

FIG. 16A is a plot from a first experiment showing results based on a previous calibration technique.

FIG. 16B is a plot from the first experiment showing results based on the present embodiments.

FIG. 17 is three plots from a second experiment showing results based on the present embodiments.

FIG. 18 is a plot from a third experiment showing results based on the present embodiments.

FIG. 19 is a plot from a fourth experiment showing results based on the present embodiments.

FIG. 20 is a plot from a fifth experiment showing results based on the present embodiments.

FIG. 21 is a schematic diagram illustrating an example of a system for executing functionality of the present invention.

## DETAILED DESCRIPTION

The following definitions are useful for interpreting terms applied to features of the embodiments disclosed herein, and are meant only to define elements within the disclosure.

As used within this disclosure, an "Artificial Neural Network" (ANN) represents mathematical models commonly used to solve (i) classification problems and (ii) regression problems. This document addresses using neural networks for a solving a regression problem. When used to approximate or replicate an existing response or set of data, an ANN may be referred to as a surrogate model. Surrogate models are mathematical models that may be created in different ways and may not necessarily be a neural network. Surrogate models may be regarded as maps between a set of inputs and a set of outputs. There are multiple types of ANN architectures, for example, Feed Forward Neural Network (FFNN), Recurrent Neural Network (RNN), and Transformers, among others. ANNs contain a number of internal parameters, including "weights" and "biases." The number of weights and biases depend on the size of the ANN and can range from tens to trillions of parameters.

As used within this disclosure, "ANN Training" refers to a process of calibrating an ANN. Regardless of the ANN architecture, the internal ANN parameters (i.e., the weights and biases) need to be calibrated before the ANN can actually be used in a meaningful way. While this calibration process is referred to in the ANN literature as "training," this ANN training process is conceptually similar to constitutive response model calibration. The ANN training also uses numerical minimization algorithms that modify the internal ANN parameters (i.e., the weights and biases) until the ANN prediction matches to a certain level of accuracy certain data that it needs to replicate.

As used within this disclosure, "Bayesian minimization" refers to a design parameter improvement and optimization framework intended for expensive "black box"- type objective functions (e.g., a time consuming simulation). The main goal of a Bayesian minimizer is to get the best possible set of design parameters with a relatively small number of objective function evaluations (for example, less than 100 objective function evaluations). The method relies on (i) creating and using a response surface for approximating the objective function and (ii) an acquisition function for predicting the next sampling point to be attempted with the expensive objective function. The method involves evaluating an "uncertainty" measure when using the response surface for approximating the expensive objective function.

As used within this disclosure, "Long Short-Term Memory Neural Networks (LSTM - NN)" refers to a particular category of recurrent neural networks. There exist different variants of LSTM neural networks in the literature. LSTM neural networks may be architected as a stack of two or more LSTM cells.

As used herein, a "history output" is a quantity provided by a simulation (based on user request) as a function of time, e.g., stress at a point in the finite element model as a function of time, reaction force recorded as a function of time during the simulation etc.

The embodiments herein describe methods using a long short-term memory (LSTM) based NN architecture to create surrogates models for replicating history outputs extracted from finite element simulations used during constitutive response calibration.

Reference will now be made in detail to embodiments of the present invention, examples of which are illustrated in the accompanying drawings. Wherever possible, the same reference numbers are used in the drawings and the description to refer to the same or like parts.

Exemplary embodiments of the present invention are used to accelerate the calibration of constitutive models to be employed during finite element simulations. The embodiments significantly reduce the time to identify the parameters of a constitutive response model, in particular for cases when time consuming finite element simulations are used to replicate the experimental testing. As such the embodiments provide a practical approach for scenarios where even a single simulation may take hours or more to complete.

The calibration workflow embodiments described here provide an iterative process that uses numerical minimization algorithms. The user provides experimental test data sets together with finite element models as part of the input to the calibration workflow. The finite element models are used to simulate the testing experiments. During an iterative calibration process, history output provided by the finite element simulations is compared to the corresponding experimental test data in an iterative manner until they are in agreement. This process may involve the finite element simulations being run multiple times (for example, from hundreds of times to tens of thousands times or more) and can take long time.

The embodiments provide an approach where the time to run a constitutive response model calibration may be reduced significantly by automatically employing neural networks based surrogate models used to replace the otherwise needed output from a corresponding finite element simulation. As a non-limiting example, the embodiments may create surrogate models using a particular long short-term memory based neural networks. Depending on the particular constitutive response model, the choice of neural network may be any neural network architecture or network of mathematical functions connected and/or pruned to process sequence data, including recurrent neural networks/recursive nets, feed forward neural networks and transformers of varying architectures customized/combined.

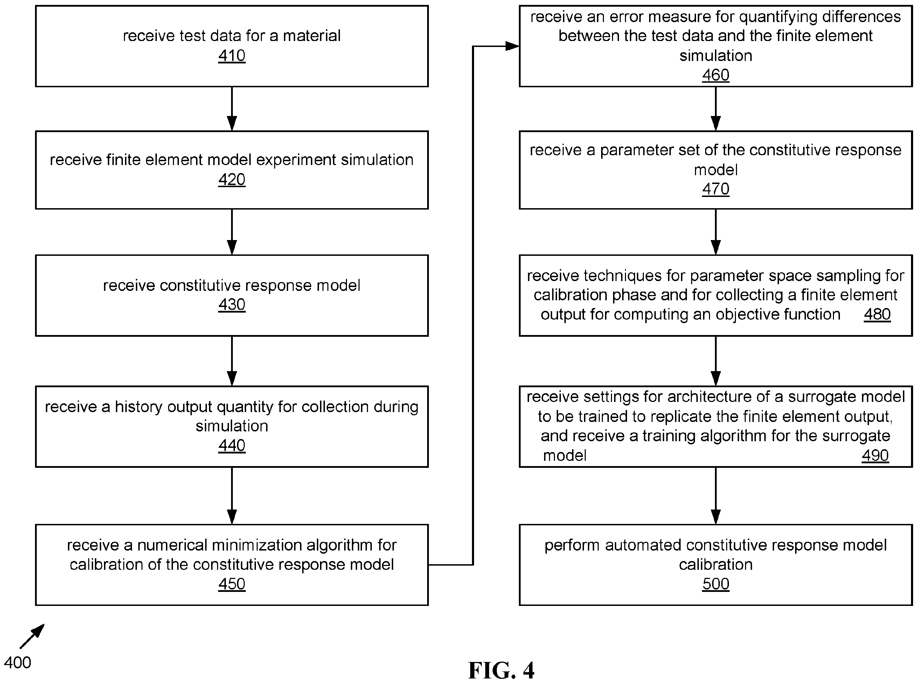

FIG. 4 is a flowchart of an exemplary material calibration workflow method. It should be noted that any process descriptions or blocks in flowcharts should be understood as representing modules, segments, portions of code, or steps that include one or more instructions for implementing specific logical functions in the process, and alternative implementations are included within the scope of the present invention in which functions may be executed out of order from that shown or discussed, including substantially concurrently or in reverse order, depending on the functionality involved, as would be understood by those reasonably skilled in the art of the present invention.

The method embodiments described here expand and enhance material calibration workflows using finite element models for example, as implemented in the 3DX Material Calibration App. While the embodiments are directed to calibration of material behaviors, alternative embodiments may also be used to calibrate the constitutive response of special elements (e.g., cohesive elements, connector elements, spring elements, and gasket elements, among others) and the constitutive interface response of contacting surfaces (such as cohesive contact, wear, friction, and others) and even fracture models.

In the following description, the terms "optimization" and "minimization" may be used interchangeably. While mathematically, optimization may refer to either a "minimization" or "maximization," it is trivial to convert between a minimization a maximization by multiplying the objective function to be minimized/maximized by (minus) -1. Further, the term "finite element (FE) response," or "FE simulation response" refers to an output quantity, which is part of the results provided by the simulation.

Under an exemplary calibration method embodiment, test data regarding a material is received, as shown by block 410. The test data may be, for example, the output from an experiment. A finite element model simulating the experiment is received, as shown by block 420. A user selection of a constitutive response model is received, as shown by block 430. A user selection of a history output quantity for collection during simulation is received, as shown by block 440. A user selection of a numerical minimization algorithm for calibration of the constitutive response model is received, as shown by block 450. A user selection of an error measure for quantifying differences between the test data and the finite element simulation is received, as shown by block 460. A user selection of a parameter of the constitutive response model is received, as shown by block 470. A user selection of a first technique for parameter space sampling during an initial calibration phase and a second technique for collecting a finite element output for computing an objective function are received, as shown by block 480. A user selection of settings regarding architecture of a surrogate model to be trained to replicate the finite element output, and a user selection of a training algorithm for the surrogate model are received, as shown by block 490. An automated constitutive response model calibration is performed, as shown by block 500. Each of blocks 410-500 is described in further detail below.

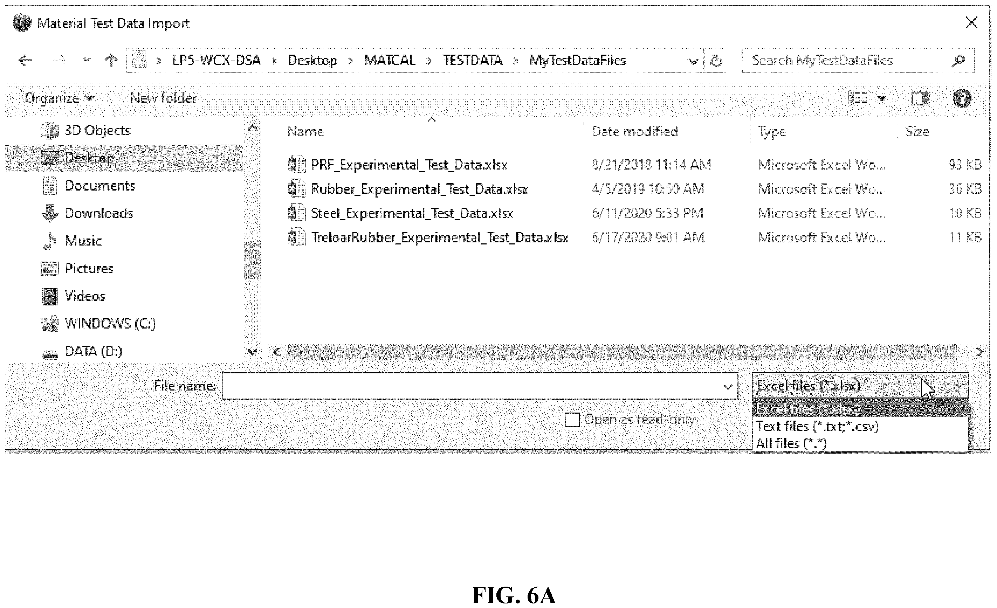

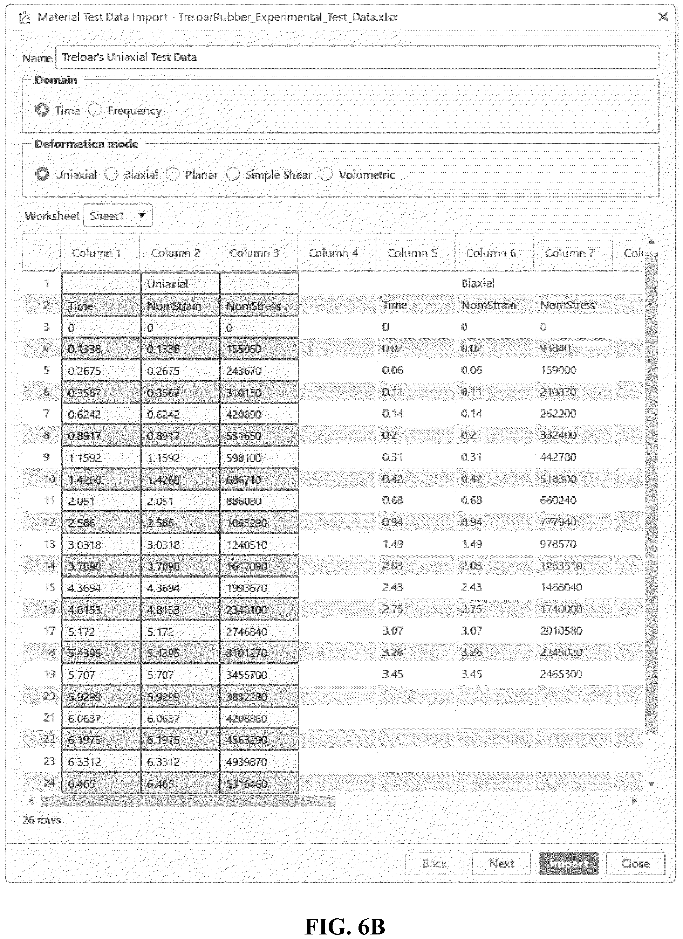

Test data regarding a material is received, as shown by block 410. A user of a simulation application provides relevant test data sets obtained previously, for example, via experiment. It should be noted, the experimental operations needed to obtain the test data sets are outside the scope of this embodiments. FIG 6A is a screenshot showing an example of importing experimental test data into the 3DX Material Calibration App. The test data sets may include, for example, stress as a function of time and deformation, force as a function of time and deformation, and/or any relevant quantity of interest recorded as a function of time if such quantity is obtainable from simulations either as direct output or based on post-processing the simulation outputs. The test data sets may be obtained in laboratories using material samples or measured using sensors placed on full parts and structures. Existing experimental techniques to obtain the test data sets as input to the method described here include, for example, universal testing machines, environmental controlled chambers, and digital image correlation, among others. FIG. 6B is a screenshot of an example of user imported experimental test data sets into the 3DX Material Calibration App. Here, the user has selected the test data of interest (from an Excel file) to be imported. In this particular example, the selected experimental test data set includes Time, Nominal Stress, and Nominal Strain. The user may import multiple test data sets from the same file or from multiple files.

The test data may be represented in tabular formats. On a computer, the test data sets may be stored, for example, in spreadsheet files, CSV (comma-separated value) lists, or even text files. For example, the 3DX Material Calibration App allows the user to import the test data sets from spreadsheet files, CSV files and TXT files. Multiple test data sets may be stored in a single file or in separate files. While this embodiment describes and depicts the 3DX Material Calibration App, alternative embodiments may involve other platforms, for example (but not restricted to) Abaqus/CAE, or invocation via a command line prompt.

Once a test data set is imported inside the 3DX Material Calibration App, the user may optionally edit the content of a test data set as needed to suit the calibration purpose. These editing operations may include removing duplicate data points, reducing the number of test data points by decimation or deletion, smoothing the test data sets to eliminate noise in the test data sets, regularization of the test data sets (regenerating the data at equally spaced intervals).

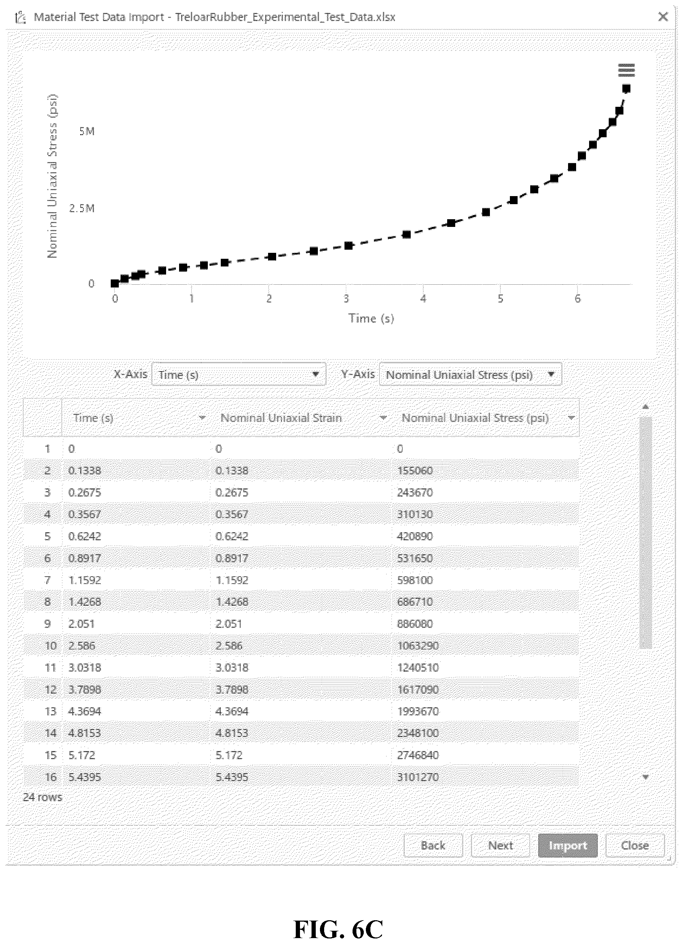

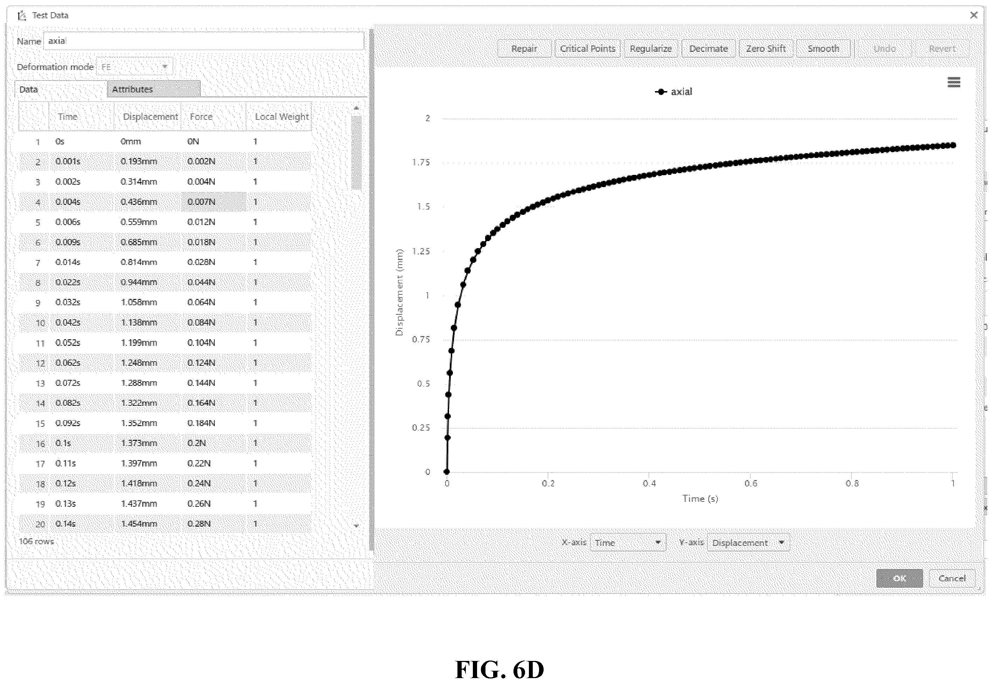

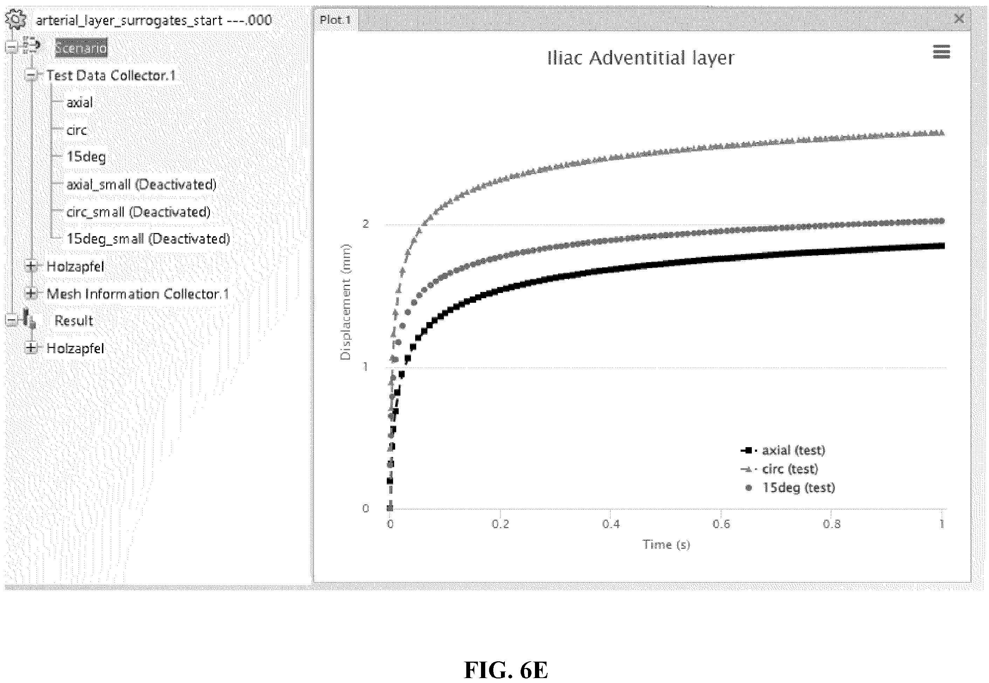

FIG. 6C shows a plot showing selected test data during the import process, here plotting experimental Nominal Stress as a function of Time (recorded during a prior experiment). FIG. 6D shows imported experimental test data for editing and display after import into the app. Here, the user may manually delete the points or can use one of the available operations (e.g., decimation, smoothing, regularization) to improve the quality of the test data set prior to the test data set being used for calibration. FIG. 6E shows multiple experimental test data sets plotted inside the app. After import, the user may choose which test data sets to be used for a calibration.

A finite element model simulating the experiment is received, as shown by block 420 (FIG. 4). The user creates and provides finite element models as input to simulate the laboratory experiments. A calibration may use one or more finite element models simultaneously. The creation process of the finite element models is not part of this invention and needs to be done in advance. Existing pre-processing software lets the user create finite element models. For example, the finite element models may be created in the 3DX Mechanical Scenario App or in Abaqus/CAE.

Exemplary formats used to describe a finite element model in the context of the embodiment include the Abaqus INPUT file format (.inp) and the (internal) 3DX simulation object format. Abaqus INPUT file format (.inp) is a text representation of the finite element models for the Abaqus software, which is publicly documented. Existing Translators between different finite element model formats may be used, for example, to translate a finite element model generated in a format specific to a certain commercial software to Abaqus INPUT file format (.inp). For example, Abaqus INPUT files may be translated from various commercial (non-Simulia) software, as shown in Table 1:

Table 1

| Commercial Software | Supported file formats for conversion into Abaqus INP format |
| --- | --- |
| Nastran | The Nastran data in a file with the extension .bdf, .dat, .nas, .nastran, .blk, or .bulk. |
| Ansys | ANSYS blocked coded database files (.cdb) |
| PAM-CRASH | The translator requires an input file created by PAM-CRASH Version 2002 or later. The input file can have any name and extension. |
| RADIOSS | The translator requires an input file in block format created by RADIOSS Version 4.4 or 5.1. The input file can have any name and an optional extension. |

Reciprocally, the Abaqus INPUT file (.inp) may be converted to other (non-DS/Simulia) formats.

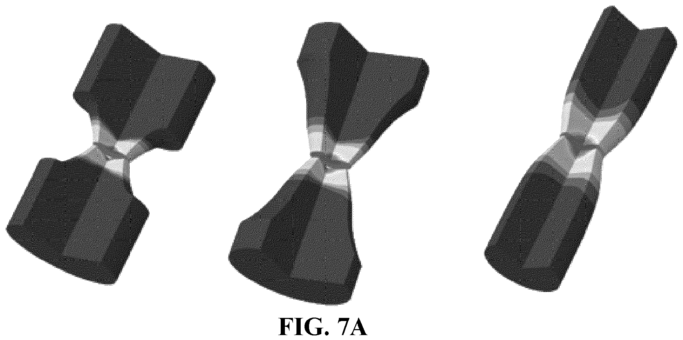

FIG. 7A shows an example of a set of three finite element models used together during the same calibration of a plasticity with damage constitutive response model (finite element meshes not shown in this figure).

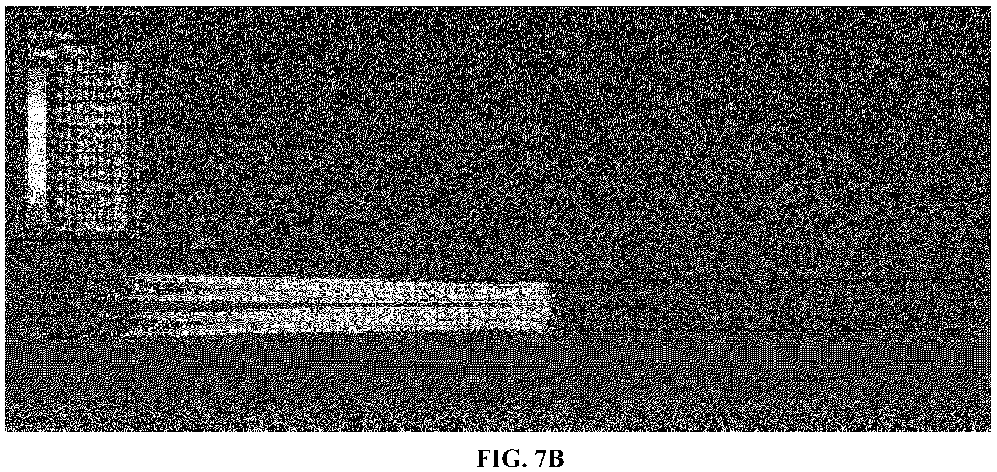

FIG. 7B shows an example of an Abaqus finite element model used for simulating a double cantilever beam experiment. This type of FE model may be used for calibrating a cohesive interface constitutive response.

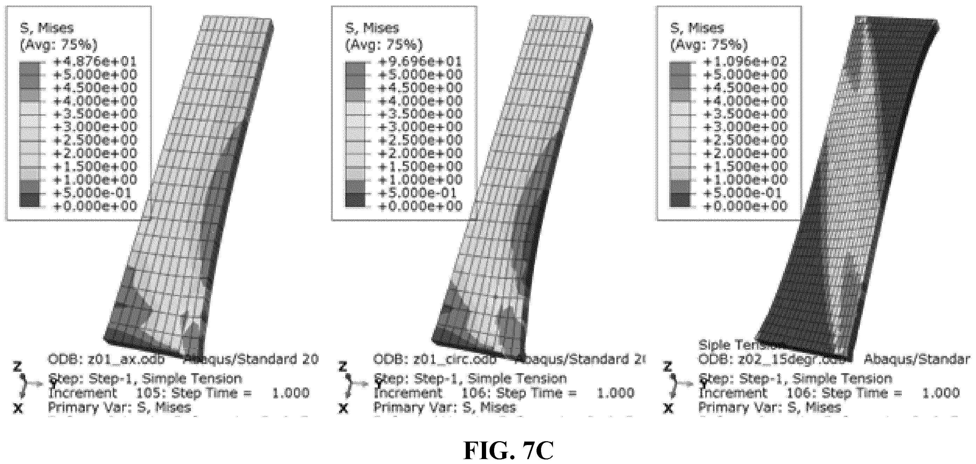

FIG. 7C shows an example of a set of three finite element models used together during the same calibration of an anisotropic hyper elastic constitutive response. Anisotropic hyper elastic responses may be used (among other applications) for modeling the response of human tissue (e.g., cardiac tissue) in a FE simulation.

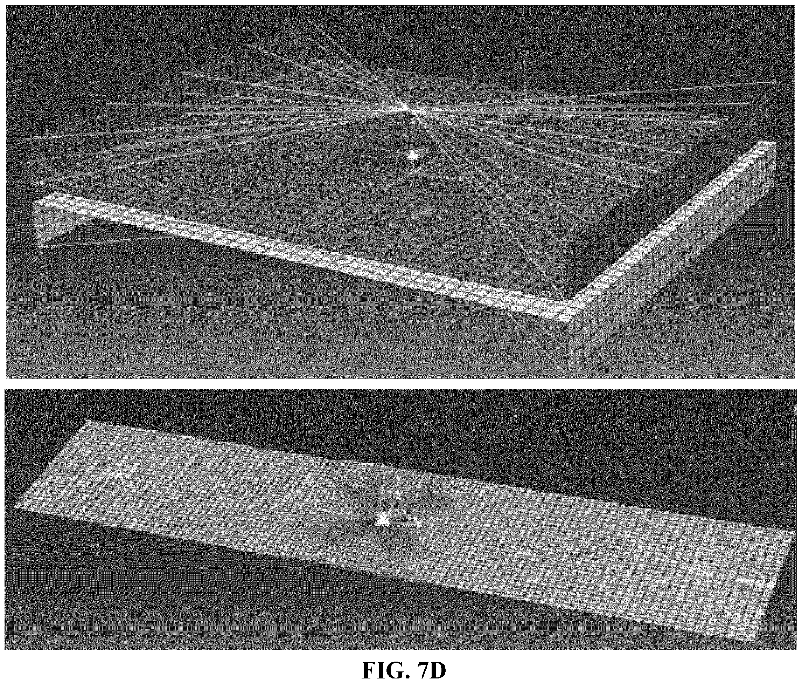

FIG. 7D shows an exemplary set of two finite element models used together for the calibration of a connector behavior, for example, a constitutive response model specific to connector elements. Here, a connector element refers to an element used to model connections between parts such as clinched joints, self-piercing rivets, spot welds, among others.

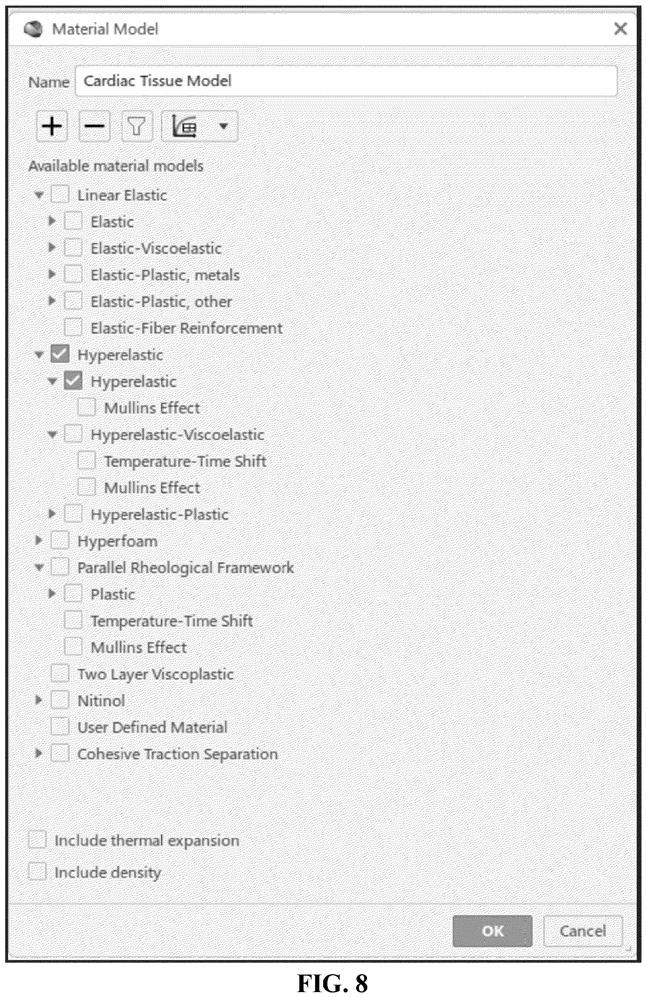

A user selection of a constitutive response model is received, as shown by block 430 (FIG. 4). The user selects the constitutive response model of interest (available, for example, in Abaqus or on the 3DX platform). In the context of this embodiment, a constitutive response model may be used in the sense of material behavior (e.g., elastic, hyper elastic, viscoelastic, plastic material behavior). However, the constitutive response model here may also refer to a mathematical model used to describe the interaction between contacting surfaces, for example, cohesive or adhesive interaction models, friction between surfaces, wear, and others. A constitutive response model may also imply a special mathematical model used to define the behavior of special types of finite elements, such as connector elements, spring elements, and cohesive elements, among others. For example, cohesive elements may be used to simulate fracture and delamination. The constitutive response for a cohesive element may be represented by the traction-separation law. FIG. 8 shows an exemplary list of constitutive response models for user selection. Each constitutive response model has a specific set of parameters that may be calibrated using relevant experimental test data sets. A mathematical description of each of these constitutive models is provided in the software documentation.

A user selection of a history output quantity for collection during simulation is received, as shown by block 440 (FIG. 4). For each finite element model to be used during calibration, the user may specify one or more history output quantities to be collected during the simulation. The history output refers to output stored as a function of time at particular model location or for the entire model. Each experimental test data quantity may be used together with a corresponding quantity obtained from one or more collected FE simulation history outputs to define an objective function.

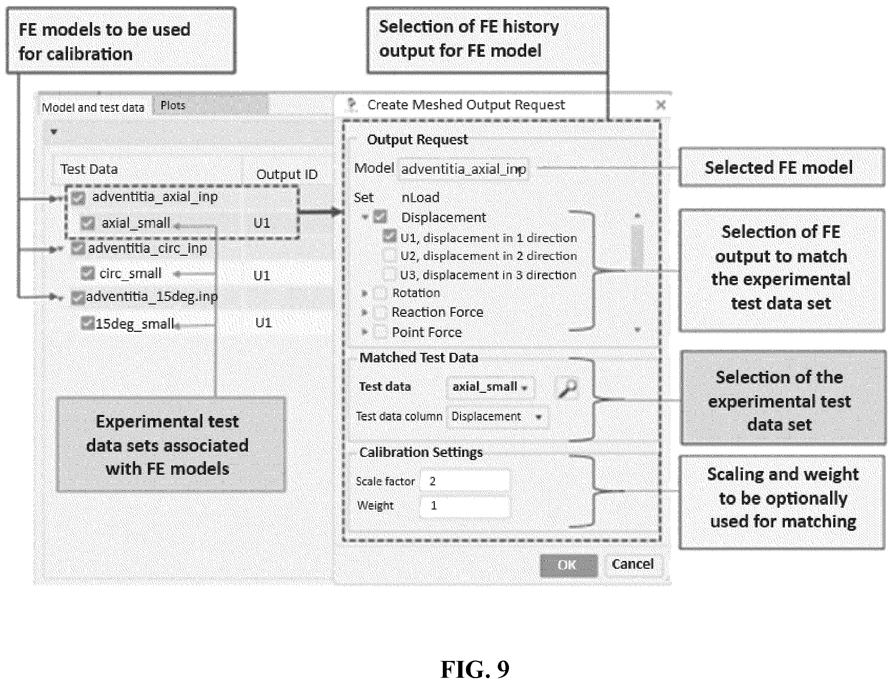

FIG. 9 is an annotated screenshot showing an exemplary calibration setup using three FE models. For each FE model, the user may select one or more FE outputs to be matched during calibration against a corresponding experimental quantity contained in one of the imported test data sets. FIG. 9 indicates that for one of the FE models, named "adventitia_axial.inp" the user want to extract during the FE simulation the FE history output representing the displacement U1 at a nodal location defined by nodal set named "nLoad". During calibration, this FE output (U1) is to be matched against the experimental displacement values extracted from an imported test data set named "axial_small". In FIG. 9, the user indicated that the FE output needs to be scaled first by a factor of 2 before matching. The error measure between the scaled FE output and the experimental quantity may be weighted by a user specified weight value.

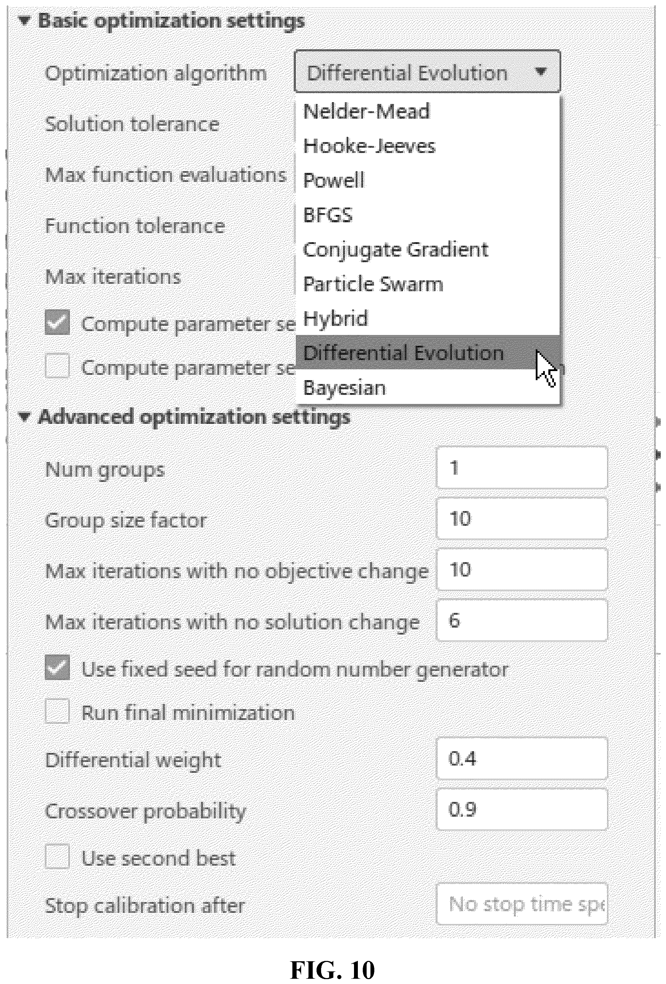

A user selection of a numerical minimization algorithm for calibration of the constitutive response model is received, as shown by block 450. The user selected numerical minimization algorithm is used during the calibration of the constitutive response model together with any relevant settings. FIG. 10 is a screenshot illustrating user selection of one of several available numerical minimizations algorithms to be used for the calibration. The user can modify the default settings if desired. Some settings are common to all algorithms, but some settings may be specific to individual algorithms. The terms "minimization" and "optimization" are used interchangeably in the dialog window.

A user selection of an error measure for quantifying differences between the test data and the finite element simulation is received, as shown by block 460. The user selects an error measure (or "error norm") to be used to quantify the differences between the test data sets and corresponding finite element simulation ("mean square error"). The error measure is a function used to form the objective function.

Eq. 1 illustrates how an objective function $F(\boldsymbol{X})$ may be obtained for a generic calibration case that employs multiple finite element (FE) models. For each FE model, an arbitrary number of history outputs may be collected from the simulations. The collected output may be combined using a post-processing function. The values provided by the post-processing function are compared to the experimentally recorded values of a corresponding test data quantity using a user indicated error measure. The error measure is a function that quantifies the differences between two sets of values, in this case experimental values vs. simulation values. Examples of error measures are mean square error, mean absolute error, coefficient of determination etc. The value returned by the error measure may be optionally scaled by a user assigned weight. A penalty value may be added to the objective function to account for failed finite element simulations (for example, due to convergence issues), various constraints violations, and others. The time $t$ in Eq. 1 may be the real time or an artificial time used to index the applied load increments.

$$F(\boldsymbol{X}) = \sum_{i=1}^{N_{FEModels}} \sum_{j=1}^{N_{TDQ}^i} w_{j;i} E\left[Q_{j;i}^{TD}(t), f_{j;i}^{FE}(q_{j1;i}^{FE}, \ldots, q_{jm;i}^{FE})\right] + Penalty \quad \text{(Eq. 1)}$$

where:

$t = time$; $\boldsymbol{X} = vector\ containing\ the\ constitutive\ parameter\ values$

$N_{FE_{Models}}$ = number of the FE models used during calibration

$N_{TDQ}^{i}$ = number of test data quantities to be fit for the ith FE model

E = error measure function (e.g., mean square error etc.)

$Q_{j;i}^{TD}(t)$ = experiment test data quantity used with the ith FE model, jth comparison

$q_{jk;i}^{FE}(t; \boldsymbol{X})$ = finite element history output obtained when running the ith FE model, used for the jth comparison, where k = 1... m

used for the jth comparison, where k = 1 ... m

$f_{j;i}^{FE}$ = post - processing function of FE history outputs $q_{jk;i}^{FE}(t; \boldsymbol{X})$

$w_{j;i}$ = weight value for jth comparison, ith FE model

For the simplest case, there is a single output quantity *(m=1)* and no output processing, i.e.,

$$f_{j;i}^{FE} = q_{j1;i}^{FE}(t; \boldsymbol{X})$$

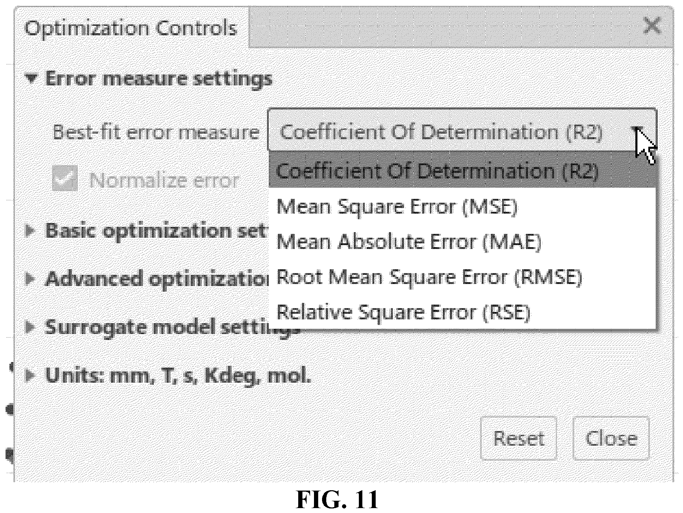

FIG. 11 is a screenshot illustrating a mechanism for the user to select an available error measure. The error measure is a function used to quantify the difference between the experimental test data and the finite element response.

A user selection of a parameter set of the constitutive response model is received, as shown by block 470. The user selects the parameters of the constitutive response model to be calibrated. Additionally, the user may optionally indicate minimum and maximum bounds for each parameter. The user specifies an initial value for each parameter. The user may optionally perform a single FE model based evaluation using the initial values of the constitutive response parameters.

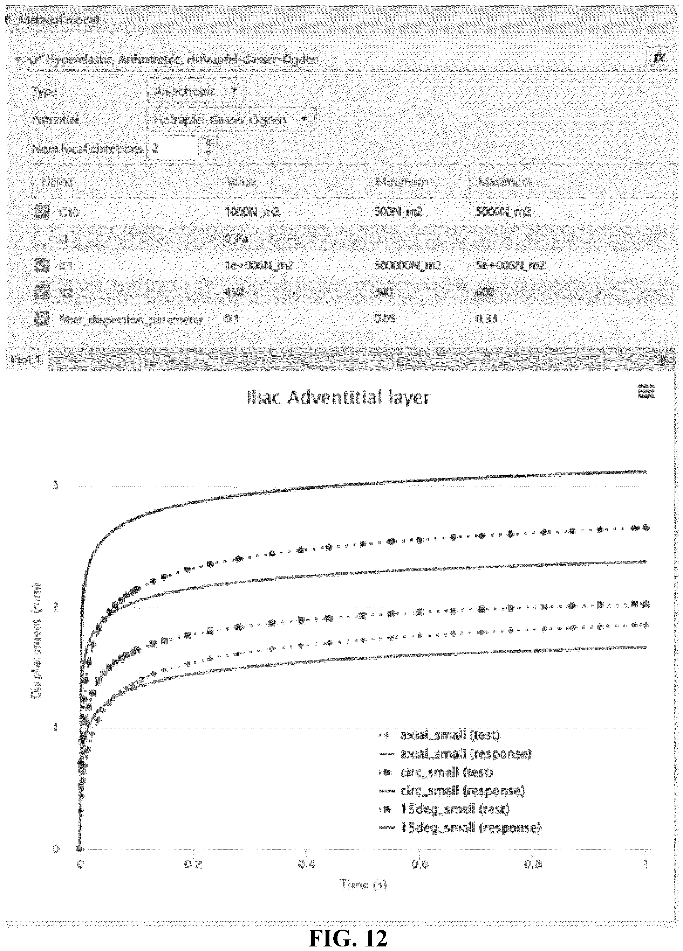

FIG. 12 shows an exemplary scenario of a user selected Anisotropic Holzapfel-Gasser-Ogden Hyperelastic constitutive material model used to simulate cardiac tissue. This constitutive response has five parameters that may be calibrated: "C10", "D", "K1", "K2" and "fiber_dispersion_parameter." In this setup, parameter D is set to a fixed known value (0 1/Pa) and not to be calibrated. The remaining parameters are to be calibrated. For each parameter to be calibrated, the user provides an initial guess and a range (defined by the minimum and maximum bounds). The plot (bottom) shows a comparison of the experimental test data vs the FE output (i.e., FE responses) when using the initial guess for the parameters to be calibrated. In this case, the initial values are far from the "real" values and the FE and experimental curves do not match very well.

The user selects a technique to be used in an initial phase of the calibration to sample the parameter space and to collect the finite element output that is needed to compute the objective function, as shown by block 480 (FIG. 4). The selected technique may be either a sampling technique (design of experiment technique) or an existing minimization algorithm. Examples of sampling techniques include Random Sampling and Latin Hypercube Sampling. A preferred minimization algorithm that may be used in this step is Bayesian minimization. Bayesian minimization is a numerical algorithm intended for expensive objective function evaluation when one tries to obtain an acceptable solution with a small number of function evaluations. Bayesian minimization may improve the sampling with points where the greatest improvement is to be expected. Optionally, the user can re-use saved FE responses evaluated during a previous calibration.

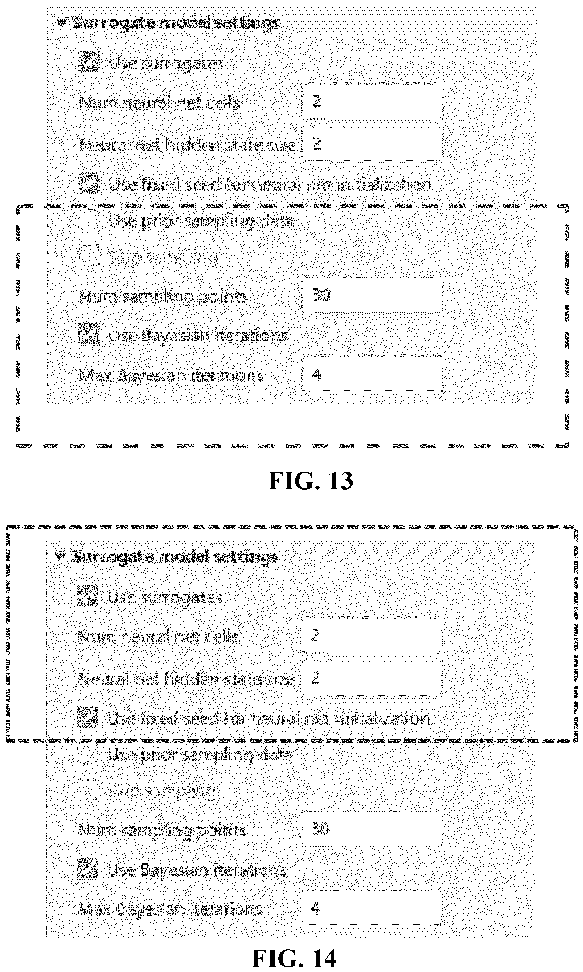

FIG. 13 illustrates a scenario where the user is presented options to modify default settings relating to initial sampling of the parameter space. As shown, the UI presents the case of a beneficial approach using Latin Hypercube method followed by Bayesian iterations. In this situation, the user may specify the number of sampling points and the number of Bayesian iterations. However, other sampling approaches (not shown) may be easily employed in alternative embodiments. The user may optionally re-use prior sampling data (collected during prior calibrations).

A user selection of settings regarding architecture of a surrogate model to be trained to replicate the finite element output, and a user selection of a training algorithm for the surrogate model are received, as shown by block 490 (FIG. 4). An architecture of interest but not limited to the approach described here is based on stack of long short-term memory (LSTM) neural networks (NN) connected to an output feed forward network. Non-default settings may include number of LSTM cells used in the NN stack, number of units per cell, and activation functions for the output forward feed NN, among others. Settings specific to NN training may include settings related to the minimization algorithms to be used for training the NNs.

FIG. 14 illustrates an example of how the user can modify the default settings controlling the NN architecture. For usability purposes, FIG. 14 shows only a small number of settings are exposed to the user in the UI. Alternative embodiments may display other features, for example, settings specific to NN training.

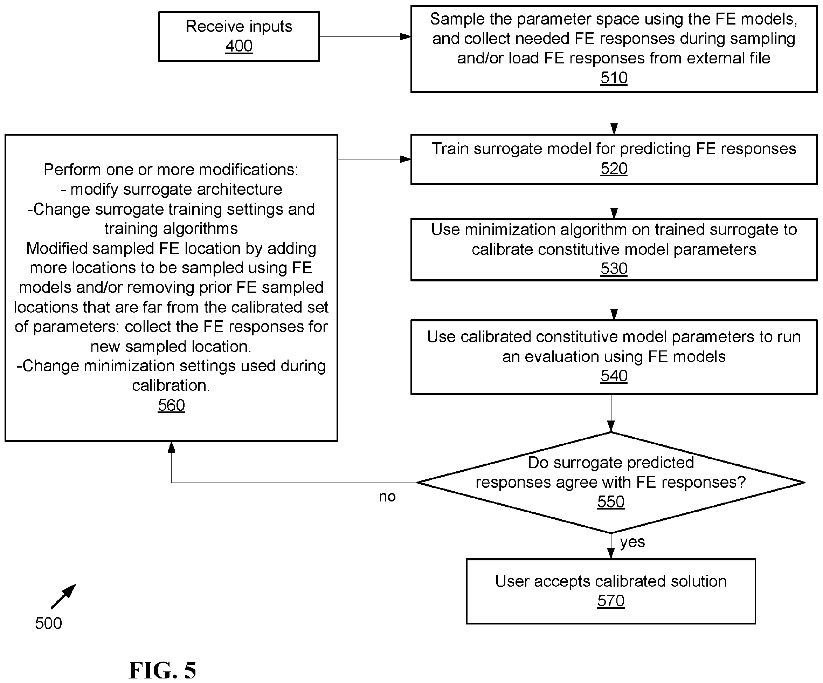

FIG. 5 is a flowchart 500 expanding on FIG. 4 block 500 illustrating the actions of the embodiment for constitutive response calibration. Block 400 represents receiving user inputs as per blocks 410-490 of FIG. 4.

The parameter space is sampled by evaluating the objective function based on using the FE models (see the description of block 480 above), and needed FE responses are collected and stored internally. In addition, prior FE responses (collected during a previous calibration) can be optionally loaded from an external file, as shown by block 510. A small number (commonly 20-40, and generally less than 100) of finite element simulations need to be performed using the finite element models provided by the user at the sampling locations. The sampling locations may be obtained using established sampling techniques such as Random Sampling or Latin Hypercube Sampling. A particularly advantageous minimization algorithm that can be used in this step (for obtaining sampling locations) is Bayesian minimization. The FE history output quantities of interest provided by the simulations (i.e., used to evaluate the objective function) are collected phase and stored internally. The collected FE history output quantities may be saved, for example to disk, and may be re-used for subsequent surrogate based calibration (based on user selection).

One or more neural networks are trained (using typical NN training approaches relying on numerical minimization algorithms such as Stochastic Gradient Descent, Adam, BFGS etc.) in order to replicate accurately the FE output of interest without the need to run the finite element simulations, as shown by block 520. This phase is important, as, depending on the actual neural network architecture, the surrogate model may or may not be able to replicate accurately the output from finite element simulations. In particular, a stack of LSTM cells followed by a Feed Forward NN is used to replicate individual history output. However, it is possible that other surrogate types may provide accurate results as well. Once trained, the trained neural networks are referred to as surrogate models. The user may switch between using the surrogate models (very fast evaluation) and finite element models (slow evaluation, but guaranteed accurate).

As shown by block 530, the surrogate models are substituted for the more computationally expensive finite element simulations in the iterative minimization process for providing the history output quantities that are used for computing the objective function (during the minimization). The result is a set of calibrated parameters for the selected constitutive response.

The calibrated constitutive response parameters are evaluated using the FE models (instead of the surrogates) for evaluation, as shown by block 540, to assess the accuracy of the surrogate models and to provide the user with the FE history outputs corresponding to the calibrated constitutive response parameters.

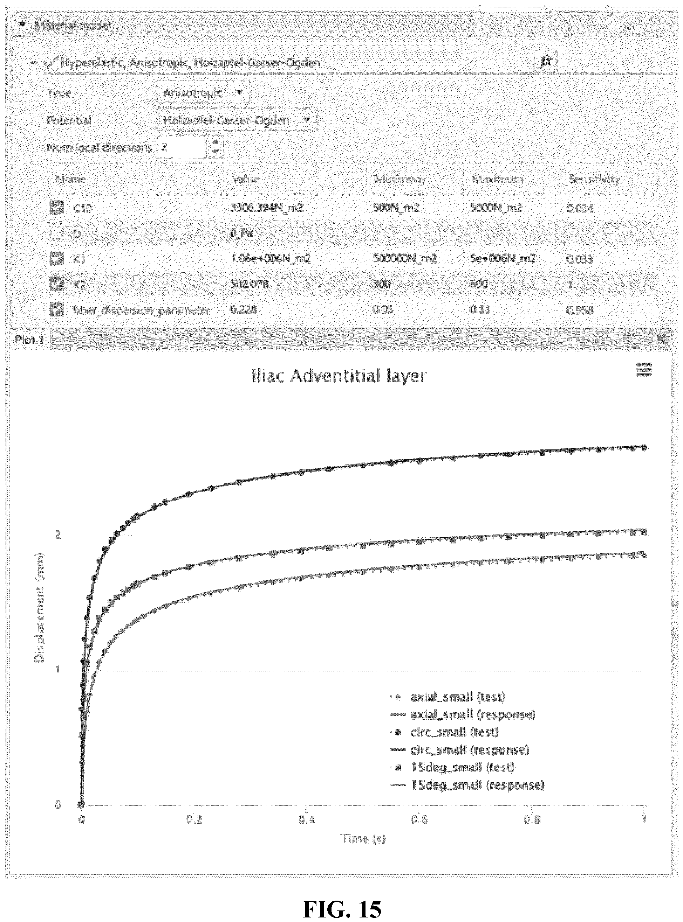

FIG. 15 is a screenshot and plot comparing the FE responses using calibrated parameters to the experimental test data. Here, the calibrated parameters of the constitutive response are reported back to the user together of the FE responses. In the example shown by FIG. 15, the calibrated parameters for the FE responses are very close to the experimental test data.

The surrogate predicted responses are compared with the FE responses, as shown by block 550 and the user is provided with the FE responses and diagnostics about the agreement between the FE responses and the surrogates prediction for the calibrated set of parameters. If the FE responses and the surrogates prediction are in agreement, the user may accept the calibrated parameters (block 570). Otherwise, the user may make one or more modifications (block 560) before re-running blocks 510-540. For example, the user may modify surrogate architecture (e.g., changing the number of LSTM cells, modifying the number of connections between cells), change surrogate training settings and training algorithms, modify sampled FE location by adding more locations to be sampled using FE models and/or removing prior FE sampled locations that are far from the calibrated set of parameters, collect the FE responses for new sampled location, and change minimization settings used during calibration.

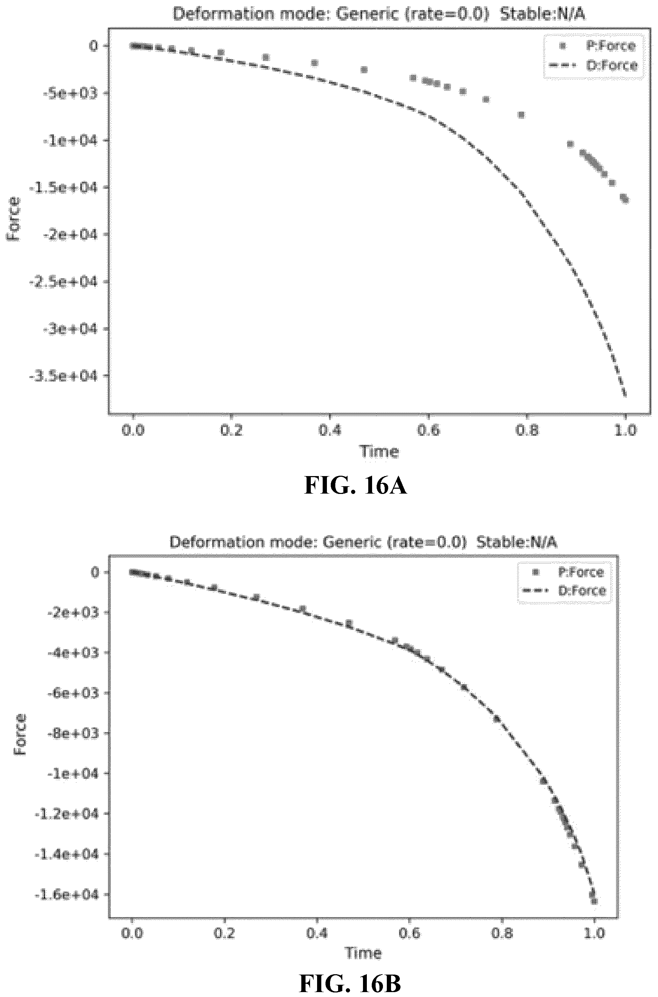

Example 1: A particular finite element model is used for the calibration of the hyperelastic YEOH model available in Abaqus. In this example, recorded Force = Function(Time) is matched between the test data and the simulation. The Nelder-Mead minimization algorithm is used. For purposes of this example, the number of the simulations is capped at 30, that is, the minimization is stopped after only 30 objective function evaluations, where each objective evaluation includes the finite element model being run once. As shown by FIG. 16A, using the pre-existing calibration approach, the minimization algorithm is not able to find a set of material parameters that would provide a good match between the simulation results and the test data set. Instead, the data collected during the 30 finite element simulation is used to train a NN-based surrogate model that predicts the force response as a function of time and material parameter values. Once trained, the surrogate model is automatically used instead of the finite element model during the minimization process. The evaluation time of the surrogate model is negligible (order of milliseconds) in comparison to the time needed to run the finite element model once. Therefore, when using the surrogate model, the minimization can use a large number of objective evaluations and the minimization can find a set of parameter values that provide a good match to the experimental data (FIG. 16B). The coefficient of determination, $R2$ is used to quantify the fit. The coefficient of determination has a maximum value $R2=1$ if the compared curves match perfectly. (The smaller the $R2 < 1$, the worse the fit is.)

As shown by FIG. 16B 30 objective evaluations are run using the collected simulation results to train a LSTM - NN surrogate to predict the Force=Function(Time, Material parameters). The trained surrogate model is then automatically used during the calibration instead of the finite element model. At the end of the calibration, using the final material properties we perform a final simulation. The simulation results (dashed line curve) does match the test data (dots).

Goodness to fit, using the coefficient of determination is R2 = 0.99

For a second example, three separate finite element models are used to calibrate a HOLZAPFEL-GASSER-OGDEN anisotropic hyperelastic material model (in Abaqus). This material model is sometimes used for simulating the behavior of cardiac tissue. Here, a separate NN surrogate model is trained for replicating the Displacement = Function(Time, Material properties) for each of the three finite element models.

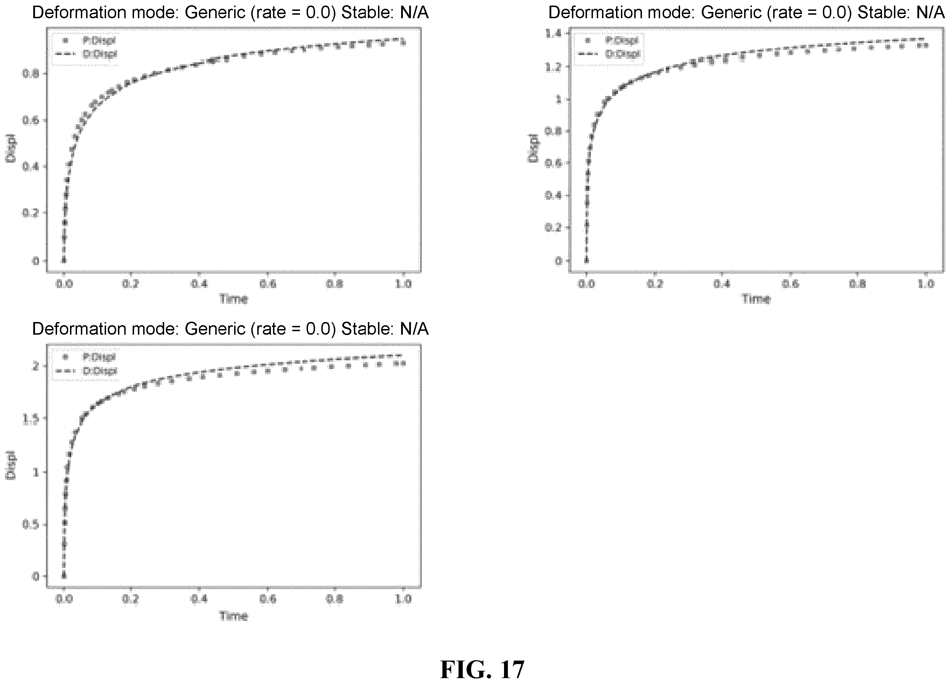

As shown by FIG. 17, 30 objective evaluations were run, and the collected simulation results were used to train a LSTM - NN surrogate to predict the Displacement=Function(Time, Material parameters). The trained surrogate models are then automatically used during the calibration instead of the finite element models. At the end of the calibration, using the final material properties we perform a final simulation using the finite element models. The simulation results (dashed line) are in good agreement with the test data (dots). Goodness to fit, using the coefficient of determination R2 > 0.99 for all 3 data sets.

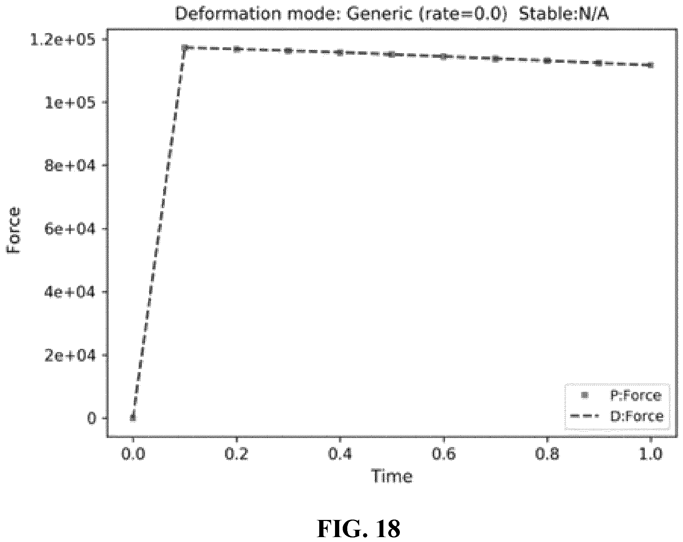

In a third example, a single finite element model is used to calibrate the friction coefficient needed by the Coulomb friction model used to describe the interaction between two contacting parts. As shown by FIG. 18, 30 objective evaluations were run, and the collected simulation results were used to train a LSTM - NN surrogate to predict the Force=Function(Time, Friction coefficient). The trained surrogate model was used during the calibration instead of the finite element model. At the end of the calibration, a final simulation was performed using the final value for the friction coefficient. The simulation results (dashed line) matches the test data (dots). Goodness to fit, using the coefficient of determination R2 > 0.999.

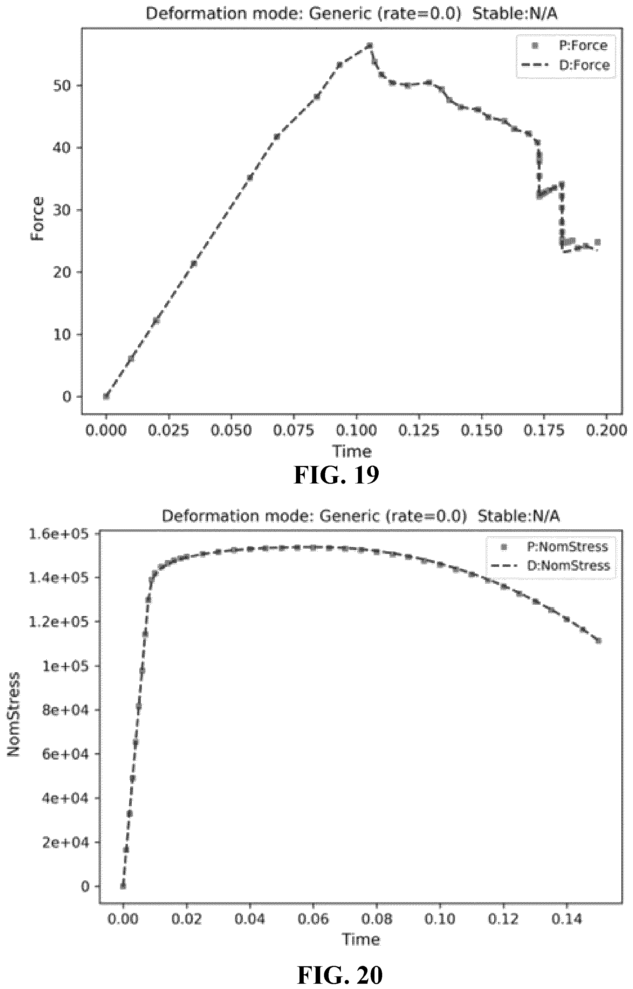

In a fourth example, a single finite element model is used to calibrate the parameters used by a fracture criterion used to crack initiation and propagation at the interface between two bonded parts. In this example, 30 objective evaluations were run, and the collected simulation results were used to train a LSTM - NN surrogate to predict the Force=Function(Time, Fracture criterion parameters). The trained surrogate model is then automatically used during the fracturing model calibration instead of the finite element model. At the end of the calibration, using the final value for the fracture criterion parameters we perform a final simulation. The simulation results (blue curve) does match the test data (red dots). Goodness to fit, using the coefficient of determination R2 > 0.87, as shown by FIG. 19.

In a fifth example, example, a single finite element model is used to calibrate the Johnson-Cook plasticity model in Abaqus/Standard. Here, 30 objective evaluations were run, and the collected simulation results were used to train a LSTM - NN surrogate to predict the Nominal Stress=Function(Time, Material model parameters). The trained surrogate model was then automatically used during the material behavior model calibration instead of the finite element model. At the end of the calibration, using the final value for the material model parameters we perform a final simulation. The simulation results (blue curve) are in agreement with the test data (red dots). Goodness to fit, using the coefficient of determination R2 = 0.999, as shown by FIG. 20.

The existing literature provides examples of Neural Networks being used as a new constitutive response models with the ANN model replacing an existing classical material model. For example, a neural network model may be used to model a hyperplastic material. This type of application for neural networks has been under development in the recent years. However, this is not how the Neural Networks are used in the context of the foregoing embodiments.

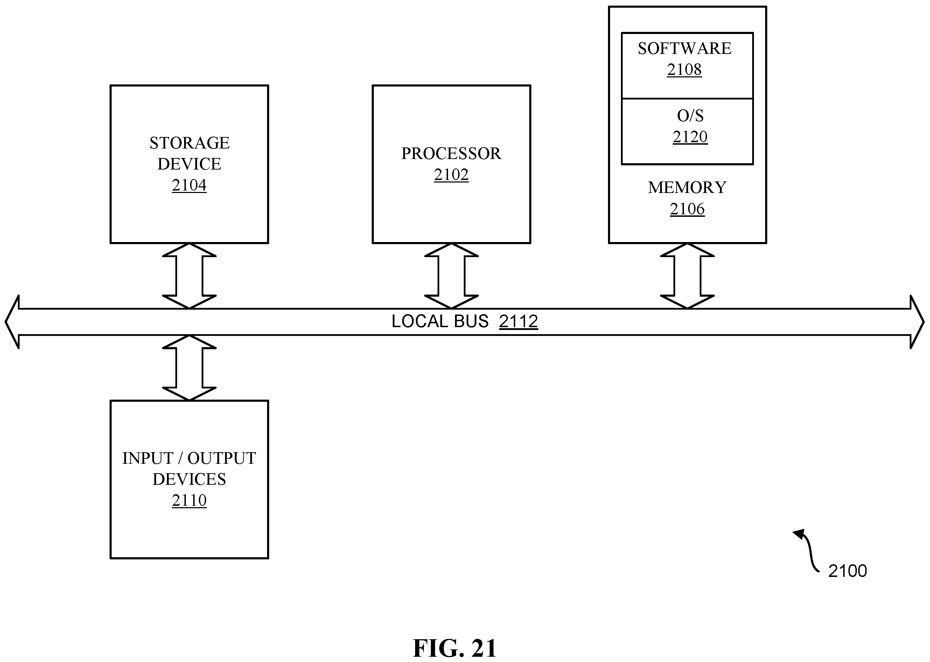

The present system for executing the functionality described in detail above may be a computer, an example of which is shown in the schematic diagram of FIG. 21. The system 2100 contains a processor 2102, a storage device 2104, a memory 2106 having software 2108 stored therein that defines the abovementioned functionality, input, and output (I/O) devices (or peripherals), and a local bus, or local interface 2112 allowing for communication within the system 2100. The local interface 2112 may be, for example but not limited to, one or more buses or other wired or wireless connections, as is known in the art. The local interface 2112 may have additional elements, which are omitted for simplicity, such as controllers, buffers (caches), drivers, repeaters, and receivers, to enable communications. Further, the local interface 2112 may include address, control, and/or data connections to enable appropriate communications among the aforementioned components.

The processor 2102 is a hardware device for executing software, particularly that stored in the memory 2106. The processor 2102 may be any custom made or commercially available single core or multi-core processor, a central processing unit (CPU), an auxiliary processor among several processors associated with the present system 2100, a semiconductor based microprocessor (in the form of a microchip or chip set), a macroprocessor, or generally any device for executing software instructions.

The memory 2106 can include any one or combination of volatile memory elements (*e.g.*, random access memory (RAM, such as DRAM, SRAM, SDRAM, *etc.*)) and nonvolatile memory elements (*e.g.*, ROM, hard drive, tape, CDROM, *etc.*). Moreover, the memory 2106 may incorporate electronic, magnetic, optical, and/or other types of storage media. Note that the memory 2106 can have a distributed architecture, where various components are situated remotely from one another, but may be accessed by the processor 2102.

The software 2108 defines functionality performed by the system 2100, in accordance with the present invention. The software 2108 in the memory 2106 may include one or more separate programs, each of which contains an ordered listing of executable instructions for implementing logical functions of the system 2100, as described below. The memory 2106 may contain an operating system (O/S) 2120. The operating system essentially controls the execution of programs within the system 2100 and provides scheduling, input-output control, file and data management, memory management, and communication control and related services.

The I/O devices 2110 may include input devices, for example but not limited to, a keyboard, mouse, scanner, microphone, *etc.* Furthermore, the I/O devices 2110 may also include output devices, for example but not limited to, a printer, display, *etc.* Finally, the I/O devices 2110 may further include devices that communicate via both inputs and outputs, for instance but not limited to, a modulator/demodulator (modem; for accessing another device, system, or network), a radio frequency (RF) or other transceiver, a telephonic interface, a bridge, a router, or other device.

When the system 2100 is in operation, the processor 2102 is configured to execute the software 2108 stored within the memory 2106, to communicate data to and from the memory 2106, and to generally control operations of the system 2100 pursuant to the software 2108, as explained above.

When the functionality of the system 2100 is in operation, the processor 2102 is configured to execute the software 2108 stored within the memory 2106, to communicate data to and from the memory 2106, and to generally control operations of the system 2100 pursuant to the software 2108. The operating system 2120 is read by the processor 2102, perhaps buffered within the processor 2102, and then executed.

When the system 2100 is implemented in software 2108, it should be noted that instructions for implementing the system 2100 may be stored on any computer-readable medium for use by or in connection with any computer-related device, system, or method. Such a computer-readable medium may, in some embodiments, correspond to either or both the memory 2106 or the storage device 2104. In the context of this document, a computer-readable medium is an electronic, magnetic, optical, or other physical device or means that can contain or store a computer program for use by or in connection with a computer-related device, system, or method. Instructions for implementing the system may be embodied in any computer-readable medium for use by or in connection with the processor or other such instruction execution system, apparatus, or device. Although the processor 2102 has been mentioned by way of example, such instruction execution system, apparatus, or device may, in some embodiments, be any computer-based system, processor-containing system, or other system that can fetch the instructions from the instruction execution system, apparatus, or device and execute the instructions. In the context of this document, a "computer-readable medium" may be any means that can store, communicate, propagate, or transport the program for use by or in connection with the processor or other such instruction execution system, apparatus, or device.

Such a computer-readable medium may be, for example but not limited to, an electronic, magnetic, optical, electromagnetic, infrared, or semiconductor system, apparatus, device, or propagation medium. More specific examples (a nonexhaustive list) of the computer-readable medium would include the following: an electrical connection (electronic) having one or more wires, a portable computer diskette (magnetic), a random access memory (RAM) (electronic), a read-only memory (ROM) (electronic), an erasable programmable read-only memory (EPROM, EEPROM, or Flash memory) (electronic), an optical fiber (optical), and a portable compact disc read-only memory (CDROM) (optical). Note that the computer-readable medium could even be paper or another suitable medium upon which the program is printed, as the program may be electronically captured, via for instance optical scanning of the paper or other medium, then compiled, interpreted, or otherwise processed in a suitable manner if necessary, and then stored in a computer memory.

In an alternative embodiment, where the system 2100 is implemented in hardware, the system 2100 may be implemented with any or a combination of the following technologies, which are each well known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), etc.

The present embodiments relate to a practical performance enhancement of an existing finite element based calibration workflow, where surrogates may be created and trained automatically during the calibration execution to replicate, for example, a specific Abaqus history output indicated by the user to be matched against a test data quantity. For example, a particular type of tested surrogate model was based on using a Neural Network that is formed from a stack of one, two or more LSTM cells followed by a Feed Forward Neural Network. Test data time and sampled parameter values of the constitutive model may be provided as input to the surrogate model, and the output was a prediction for the Abaqus history output as a function of time.

Under the present embodiments, multiple NNs (one for each test data set) may be trained independently. If any one of the NNs is not accurate, this NN may be removed from the calibration. The objective function used by the minimization algorithm may be switched on the fly between using finite element simulations and using the surrogate models during the evaluation of the objective function. Under the embodiments, parameters of the constitutive response may be sampled using different approaches. A particularly useful approach is to use Bayesian optimization iterations.

The exemplary embodiments may significantly reduce the time needed to identify the parameters of a constitutive response model when computationally expensive finite element models are used during the calibration process.

As a simple example, assuming a finite element simulation used during the calibration process needs 15 minutes to be run just once, the expected total time for a calibration that needs to run the same simulation 200 times would be expected to be about 3000 minutes. However, using the present embodiments, the same simulation may be run significantly fewer times to get acceptable results, for example, only 20-50 times instead of 200 times.

In some cases, the finite element models may take hours or more to run a single time and the calibration may need to run the same simulation thousands of times. In such a case, the embodiments may provide the only practical way to calibrate the constitutive response model.

Alternative embodiments may employ a Transformers Neural Network. The Transformers neural network architecture eschews recurrence and instead rely solely on attention mechanism, which allows the network to capture dependencies across variable length sequences without regard to their distance in the input or output sequences. Transformers may be used in a manner similar to LSTMs and LSTM-NNs to create surrogate models for replicating history outputs extracted from finite element simulations used during constitutive response calibration with history dependent material responses.

It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims and their equivalents.

## Claims

1. A computer-implemented method for reducing a time to run a constitutive response model calibration by automatically employing neural networks based surrogate models in place of output from a corresponding finite element simulation, comprising steps of:

   receiving test data regarding a material, wherein the test data comprises output from an experiment;
   receiving a finite element model simulating the experiment;
   receiving a user selection of a constitutive response model;
   receiving a user selection of a history output quantity for collection during simulation;
   receiving a user selection of a numerical minimization algorithm for calibration of the constitutive response model;
   receiving a user selection of an error measure for quantifying differences between the test data and output of the finite element simulation;
   receiving a user selection of parameters of the constitutive response model;
   receiving a user selection of a technique for parameter space sampling during an initial calibration phase when a finite element output for computing an objective function is collected;
   receiving a user selection of settings regarding architecture of a surrogate model to be trained to replicate the collected finite element output;
   receiving a user selection of a training algorithm for the surrogate model; and
   performing the automated constitutive response model calibration.

2. The computer-implemented method of claim 1, wherein the surrogate model is created with a long short-term memory neural network.

3. The computer-implemented method of claim 1 or 2, wherein the received test data includes at least one of the group of stress data as a function of time and/or deformation, and force as a function of time and/or deformation.

4. The computer-implemented method of any one of claims 1 to 3, further comprising:

   performing a plurality of finite element simulations using the finite element model at a location determined by the selected sampling or minimization algorithm;
   collecting a finite element history output from the plurality of simulations; and
   training a neural network to approximate the finite element output in lieu of the finite element simulations.

5. The computer-implemented method of claim 4, further comprising:

   replacing the finite element simulation with a surrogate model comprising the trained neural network; and
   generating a surrogate history output based on the surrogate model to produce a set of calibrated parameters for a selected constitutive response.

6.  The computer-implemented method of claim 5, further comprising:

    reverting to using the finite element model; and
    evaluating the results of the finite element model against the surrogate model.

7.  The computer-implemented method of claim 4, further comprising storing the finite element history.

8.  The computer-implemented method of claim 4, wherein the neural network comprises a stack of LSTM cells followed by a feed forward neural network.

9.  The computer-implemented method of any one of claims 1 to 8, wherein the user selection of the parameter of the constitutive response model indicate minimum and/or maximum bounds for the parameter.

10. The computer-implemented method of any one of claims 1 to 9, wherein the technique for parameter space sampling comprises Bayesian minimization.

11. The computer-implemented method of claim 10, further comprising a step of receiving a user specified number of Bayesian iterations.

12. The computer-implemented method of any one of claims 1 to 11, further comprising a step of receiving a user specified number of sampling points.

13. A computer-program comprising instructions which, when the program is executed by a computer, cause the computer to carry out the method of any one of claims 1 to 12.

14. A computer readable storage medium having recorded thereon the computer program of claim 13.

15. A system comprising a processor coupled to a memory, the memory having recorded thereon the computer program of claim 13.

FIG. 1A

Simple shear test

**FIG. 1B**

**FIG. 2**

FIG. 3

EP 4 617 941 A1

receive test data for a material
410

receive finite element model experiment simulation
420

receive constitutive response model
430

receive a history output quantity for collection during simulation
440

receive a numerical minimization algorithm for calibration of the constitutive response model
450

receive an error measure for quantifying differences between the test data and the finite element simulation
460

receive a parameter set of the constitutive response model
470

receive techniques for parameter space sampling for calibration phase and for collecting a finite element output for computing an objective function    480

receive settings for architecture of a surrogate model to be trained to replicate the finite element output, and receive a training algorithm for the surrogate model    490

perform automated constitutive response model calibration
500

400

**FIG. 4**

```
┌─────────────────────┐        ┌──────────────────────────────────────────┐
│   Receive inputs    │───────▶│  Sample the parameter space using the FE   │
│        400          │        │  models, and collect needed FE responses   │
└─────────────────────┘        │  during sampling and/or load FE responses  │
                               │          from external file                 │
                               │                 510                         │
                               └──────────────────────────────────────────┘
                                                   │
                                                   ▼
┌─────────────────────────────┐     ┌──────────────────────────────────────┐
│ Perform one or more         │     │ Train surrogate model for predicting   │
│ modifications:              │────▶│         FE responses                   │
│ - modify surrogate          │     │              520                       │
│   architecture              │     └──────────────────────────────────────┘
│ -Change surrogate training  │                    │
│  settings and training      │                    ▼
│  algorithms                 │     ┌──────────────────────────────────────┐
│ Modified sampled FE location│     │ Use minimization algorithm on trained  │
│ by adding more locations to │     │ surrogate to calibrate constitutive    │
│ be sampled using FE models  │     │         model parameters               │
│ and/or removing prior FE    │     │              530                       │
│ sampled locations that are  │     └──────────────────────────────────────┘
│ far from the calibrated set │                    │
│ of parameters; collect the  │                    ▼
│ FE responses for new        │     ┌──────────────────────────────────────┐
│ sampled location.           │     │ Use calibrated constitutive model      │
│ -Change minimization        │     │ parameters to run an evaluation using   │
│ settings used during        │     │         FE models                      │
│ calibration.                │     │              540                       │
│           560               │     └──────────────────────────────────────┘
└─────────────────────────────┘                    │
              ▲                                     ▼
              │                        ╱◇─────────────────────◇╲
              │              no       ◇   Do surrogate predicted  ◇
              └──────────────────────◇  responses agree with FE    ◇
                                      ◇      responses?             ◇
                                       ◇         550              ◇
                                        ╲◇─────────────────────◇╱
                                                   │ yes
                                                   ▼
                                      ┌──────────────────────────────┐
                                      │ User accepts calibrated        │
                                      │        solution                │
                                      │          570                   │
                                      └──────────────────────────────┘
```

500

**FIG. 5**

**FIG. 6A**

Material Test Data Import - TreloarRubber_Experimental_Test_Data.xlsx ✕

Name | Treloar's Uniaxial Test Data

**Domain**

◉ Time ○ Frequency

**Deformation mode**

◉ Uniaxial ○ Biaxial ○ Planar ○ Simple Shear ○ Volumetric

Worksheet | Sheet1 ▾

| | Column 1 | Column 2 | Column 3 | Column 4 | Column 5 | Column 6 | Column 7 | Col |
|----|----------|----------|----------|----------|----------|----------|----------|-----|
| 1 | | Uniaxial | | | | Biaxial | | |
| 2 | Time | NomStrain | NomStress | | Time | NomStrain | NomStress | |
| 3 | 0 | 0 | 0 | | 0 | 0 | 0 | |
| 4 | 0.1338 | 0.1338 | 155060 | | 0.02 | 0.02 | 93840 | |
| 5 | 0.2675 | 0.2675 | 243670 | | 0.06 | 0.06 | 159000 | |
| 6 | 0.3567 | 0.3567 | 310130 | | 0.11 | 0.11 | 240870 | |
| 7 | 0.6242 | 0.6242 | 420890 | | 0.14 | 0.14 | 262200 | |
| 8 | 0.8917 | 0.8917 | 531650 | | 0.2 | 0.2 | 332400 | |
| 9 | 1.1592 | 1.1592 | 598100 | | 0.31 | 0.31 | 442780 | |
| 10 | 1.4268 | 1.4268 | 686710 | | 0.42 | 0.42 | 518300 | |
| 11 | 2.051 | 2.051 | 886080 | | 0.68 | 0.68 | 660240 | |
| 12 | 2.586 | 2.586 | 1063290 | | 0.94 | 0.94 | 777940 | |
| 13 | 3.0318 | 3.0318 | 1240510 | | 1.49 | 1.49 | 978570 | |
| 14 | 3.7898 | 3.7898 | 1617090 | | 2.03 | 2.03 | 1263510 | |
| 15 | 4.3694 | 4.3694 | 1993670 | | 2.43 | 2.43 | 1468040 | |
| 16 | 4.8153 | 4.8153 | 2348100 | | 2.75 | 2.75 | 1740000 | |
| 17 | 5.172 | 5.172 | 2746840 | | 3.07 | 3.07 | 2010580 | |
| 18 | 5.4395 | 5.4395 | 3101270 | | 3.26 | 3.26 | 2245020 | |
| 19 | 5.707 | 5.707 | 3455700 | | 3.45 | 3.45 | 2465300 | |
| 20 | 5.9299 | 5.9299 | 3832280 | | | | | |
| 21 | 6.0637 | 6.0637 | 4208860 | | | | | |
| 22 | 6.1975 | 6.1975 | 4563290 | | | | | |
| 23 | 6.3312 | 6.3312 | 4939870 | | | | | |
| 24 | 6.465 | 6.465 | 5316460 | | | | | |

26 rows

Back | Next | Import | Close

**FIG. 6B**

**FIG. 6C**

**FIG. 6D**

**FIG. 6E**

**FIG. 7A**

FIG. 7B

**FIG. 7C**

FIG. 7D

Material Model      ✕

Name | Cardiac Tissue Model

➕ ➖ ▽ ▣ ▾

Available material models

▾ ☐ Linear Elastic
   ▸ ☐ Elastic
   ▸ ☐ Elastic-Viscoelastic
   ▸ ☐ Elastic-Plastic, metals
   ▸ ☐ Elastic-Plastic, other
   ☐ Elastic-Fiber Reinforcement
▾ ☑ Hyperelastic
   ▾ ☑ Hyperelastic
     ☐ Mullins Effect
   ▾ ☐ Hyperelastic-Viscoelastic
     ☐ Temperature-Time Shift
     ☐ Mullins Effect
   ▸ ☐ Hyperelastic-Plastic
▸ ☐ Hyperfoam
▾ ☐ Parallel Rheological Framework
   ▸ ☐ Plastic
   ☐ Temperature-Time Shift
   ☐ Mullins Effect
☐ Two Layer Viscoplastic
▸ ☐ Nitinol
☐ User Defined Material
▸ ☐ Cohesive Traction Separation

☐ Include thermal expansion
☐ Include density

     OK    Cancel

**FIG. 8**

FIG. 9

▼ **Basic optimization settings**

Optimization algorithm | Differential Evolution ▼

- Nelder-Mead
- Hooke-Jeeves
- Powell
- BFGS
- Conjugate Gradient
- Particle Swarm
- Hybrid
- **Differential Evolution**
- Bayesian

Solution tolerance

Max function evaluations

Function tolerance

Max iterations

☑ Compute parameter se

☐ Compute parameter se

▼ **Advanced optimization settings**

| | |
|---|---|
| Num groups | 1 |
| Group size factor | 10 |
| Max iterations with no objective change | 10 |
| Max iterations with no solution change | 6 |

☑ Use fixed seed for random number generator

☐ Run final minimization

| | |
|---|---|
| Differential weight | 0.4 |
| Crossover probability | 0.9 |

☐ Use second best

Stop calibration after | No stop time spe

**FIG. 10**

**FIG. 11**

FIG. 12

▼ **Surrogate model settings**

☑ Use surrogates

Num neural net cells      2

Neural net hidden state size    2

☑ Use fixed seed for neural net initialization

☐ Use prior sampling data

☐ Skip sampling

Num sampling points      30

☑ Use Bayesian iterations

Max Bayesian iterations     4

## FIG. 13

▼ **Surrogate model settings**

☑ Use surrogates

Num neural net cells      2

Neural net hidden state size    2

☑ Use fixed seed for neural net initialization

☐ Use prior sampling data

☐ Skip sampling

Num sampling points      30

☑ Use Bayesian iterations

Max Bayesian iterations     4

## FIG. 14

EP 4 617 941 A1

FIG. 15

34

**FIG. 16A**

**FIG. 16B**

**FIG. 17**

**FIG. 18**

**FIG. 19**

**FIG. 20**

**FIG. 21**

Europäisches
Patentamt

European
Patent Office

Office européen
des brevets

**EUROPEAN SEARCH REPORT**

**Application Number**

EP 25 16 3787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LOGARZO HERNAN J ET AL: "Smart constitutive laws: Inelastic homogenization through machine learning", COMPUTER METHODS IN APPLIED MECHANICS AND ENGINEERING, NORTH-HOLLAND, AMSTERDAM, NL, vol. 373, 21 October 2020 (2020-10-21), XP086401414, ISSN: 0045-7825, DOI: 10.1016/J.CMA.2020.113482 [retrieved on 2020-10-21] | 1-9, 12-15 | INV. G06F30/23 G06F30/27 G06N3/044 ADD. G06F111/10 G06F119/14 |
| Y | * abstract * * section 3.2 par.1, section 3 par.1; figures 2,5 * * page 4, last paragraph * * page 5, paragraph 1 * * section 4.1.1 * * page 9, paragraph 2 * * penultimate paragraph; page 4 * * the whole document * | 10,11 | |
| | ----- | | TECHNICAL FIELDS SEARCHED (IPC) |
| Y | RAVIOLO DAVIDE ET AL: "A Bayesian sampling optimisation strategy for finite element model updating", JOURNAL OF CIVIL STRUCTURAL HEALTH MONITORING, SPRINGER BERLIN HEIDELBERG, BERLIN/HEIDELBERG, vol. 15, no. 6, 20 February 2024 (2024-02-20), pages 1585-1607, XP038342869, ISSN: 2190-5452, DOI: 10.1007/S13349-023-00759-5 [retrieved on 2024-02-20] * abstract * * the whole document * | 10,11 | G06F G06N |
| | ----- | | |
| | -/-- | | |

The present search report has been drawn up for all claims

| Place of search | Date of completion of the search | Examiner |
|---|---|---|
| Munich | 14 July 2025 | Dapp, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

| Europäisches Patentamt European Patent Office Office européen des brevets | **EUROPEAN SEARCH REPORT** | **Application Number** EP 25 16 3787 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Nheri Designsafe: "Uncertainty Quantification Training for Application to Natural Hazards Engineering - Day 1 (2/22/22)", , 23 February 2022 (2022-02-23), XP093295436, Retrieved from the Internet: URL:https://www.youtube.com/watch?v=5oWEAiC12Dg * time stamp 20:30 and onwards * * the whole document * ----- | 1,13-15 | |
| | | | TECHNICAL FIELDS SEARCHED (IPC) |

The present search report has been drawn up for all claims

| Place of search | Date of completion of the search | Examiner |
|---|---|---|
| Munich | 14 July 2025 | Dapp, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)